# EUROPEAN PATENT SPECIFICATION

(11) **EP 1 274 302 B1**
(45) Date of publication and mention of the grant of the patent: **30.06.2004**
(21) Application number: 01928591.5
(22) Date of filing: 17.04.2001
(51) Int. Cl.: A01N 1/02

(54) **CYCLOHEXANEDIOL CRYOPROTECTANT COMPOUNDS**
CYCLOHEXANDIOL KRYOPROTEKTOR VERBINDUNGEN
COMPOSES DE CYCLOHEXANEDIOL CRYOPROTECTEURS

(30) Priority: 17.04.2000 US 197669 P
(43) Date of publication of application: 15.01.2003
(73) Proprietor: Organ Recovery Systems, Inc., Des Plaines, IL 60018 (US)
(72) Inventor: BROCKBANK, Kelvin, G., M., Charleston, SC 29401 (US); TAYLOR, Michael, J., Mount Pleasant, SC 29464 (US); CAMPBELL, Lia, Hanson, Mount Pleasant, SC 29464 (US)
(74) Representative: Guerre, Dominique
(86) International application number: PCT/US2001/012465
(87) International publication number: WO 2001/078505

(56) References cited:
- WO-A-00/16618
- WO-A-99/18169
- DATABASE CHEMABS [Online] CHEMICAL ABSTRACTS SERVICE, COLUMBUS, OHIO, US; O'CONNELL, KATHLEEN M. ET AL: "Cryoprotectants for Crithidia fasciculata stored at -20.deg.. Trypanosoma gambiense and T. conorhini" retrieved from STN Database accession no. 70:103995 XP002177168 & J. PROTOZOOL. (1968), 15(4), 719-24 ,

## Description

This invention was made with government support under grant number Cooperative Agreement Number 70NANB7H3071, awarded by the Department of Commerce. The government has certain rights in the invention.

### BACKGROUND OF THE INVENTION

### 1. Field of Invention

This invention relates to particular cyclohexanediol molecules and their use as cryoprotectants.

### 2. Description of Related Art

Cryobiology may be defined as the study of the effects of temperatures of lower than normal physiologic ranges upon biologic systems. During the past half-century the fundamentals of the science of cryobiology have evolved to the point where low temperatures are now used extensively as a means to protect and preserve biological systems during enforced periods of ischemia and hypoxia. In practice, preservation is achieved using either hypothermia without freezing, or cryopreservation in which the aqueous system sustains a physical phase change with the formation of ice. Survival of cells from the rigors of freezing and thawing in cryopreservation procedures is only attained by using appropriate cryoprotective agents (CPAs) and in general, these techniques are applicable to isolated cells in suspension or small aggregates of cells in simple tissues. More complex tissues and organs having a defined architecture are not easily preserved using conventional cryopreservation techniques, which is principally due to the deleterious effects of ice formation in an organized multicellular tissue. Simply freezing cells or tissues results in dead, nonfunctional materials.

The modem era of cryobiology really began with the discovery of the cryoprotective properties of glycerol as reported by Polge et al., "Revival of Spermatazoa After Vitrification and Dehydration at Low Temperatures," Nature, 164:666 (1949). Subsequently, Lovelock et al., "Prevention of Freezing Damage to Living Cells by Dimethyl Sulfoxide," Nature, 183:1394 (1959), discovered that dimethyl sulfoxide was also a cryoprotectant, and despite the wide range of compounds now known to exhibit cryoprotective properties, it is still the most widely used compound to date.

A review of the principles of cryobiology can be found in Brockbank, Principles of Cryopreserved Venous Transplantation, Chapter 10, "Essentials of Cryobiology" (1995). A basic principle of cryobiology is that the extent of freezing damage depends upon the amount of free water in the system and the ability of that water to crystallize during freezing. Many types of isolated cells and small aggregates of cells can be frozen simply by following published procedures, but obtaining reproducible results for more complex tissues requires an understanding of the major variables involved in tissue cryopreservation. Major variables involved in tissue freezing include (1) freezing-compatible pH buffers, (2) cryoprotectant choice, concentration and administration, (3) cooling protocol, (4) storage temperature, (5) warming protocol and (6) cryoprotectant elution.

Many cryoprotectants have been discovered. See, for example, Brockbank, supra. Cryoprotectant selection for cryopreservation is usually restricted to those that confer cryoprotection in a variety of biological systems. On occasion, combinations of cryoprotectants may result in additive or synergistic enhancement of cell survival. Comparison of chemicals with cryoprotectant properties reveals no common structural features. These chemicals are usually divided into two classes: (1) intracellular cryoprotectants with low molecular weights that penetrate cells, and (2) extracellular cryoprotectants with relatively high molecular weights (greater than or equal to sucrose (342 daltons)) which do not penetrate cells. Intracellular cryoprotectants, such as glycerol and dimethyl sulfoxide at concentrations from 0.5 to 3 molar, are effective in minimizing cell damage in many slowly frozen biological systems. Extracellular cryoprotective agents such as polyvinylpyrrolidone or hydroxyethyl starch are often more effective at protecting biological systems cooled at rapid rates.

WO 99/18169 by the same inventor as the present application describes methods of inhibiting or promoting growth of ice crystals by applying to the material in which ice crystals are to be grown or inhibited a molecule comprising an aliphatic moiety bearing at least two substituents that simultaneously form hydrogen bonds with ice. See claims 1 and 2 therein. Such moieties are described to include 1,3-cyclohexanediol residue.

WO 00/16618 describes improved cryoprotectant solutions exhibiting less toxicity. See the Abstract. The description focuses on a methodology of predicting toxicity of a given cryoprotectant solution. At the middle of page 9, it is mentioned that preferred cryoprotectant solutions include a penetrating cryoprotectant, a non-penetrating cryoprotectant, and a specific ice-blocking cryoprotectant.

J. Protozool. (1968) 15(4), 719-724 (O'Connell et al.) describes numerous compounds evaluated for their suitability as cryoprotectants for non-mammalian protozoa. While some of the materials evaluated were shown to be suitable in the cryoprotection of the protozoa, including 1,4-cyclohexanediol, many were found to be unsuitable, including 1,3-cyclohexanediol.

What is still desired are improved cryoprotectant materials that increase cell viability during cryopreservation.

### SUMMARY OF THE INVENTION

It is therefore one object of the present invention to provide a cryoprotectant material that effectively protects cells during cryopreservation and achieves increased cell viability upon warming from a frozen state.

It is still a further object of the present invention to provide a cryoprotectant material that is capable of obtaining consistent and reproducible results in cryopreserving cells and tissues.

It is a still further object of the present invention to provide a cryoprotectant material that is able to work in conjunction with naturally occurring anti-freeze proteins (AFPs) to promote survival of cells after freezing in a cumulative manner.

These and other objects are achieved by the present invention, which relates to the use of newly discovered cryoprotectant compounds. In particular, the invention relates to the use of cyclohexanediol compounds, specifically the cis or trans forms of 1,3-cyclohexanediol (1,3CHD) and 1,4-cyclohexanediol (1,4CHD), and their racemic mixtures, as cryoprotectants in preserving living cells.

In the invention, cells to be cryopreserved are protected against the effects of cryopreservation by bringing the cells into contact with a cryopreservation composition containing at least one cyclohexanediol compound, and subsequently reducing the temperature of the cells to the cryopreservation temperature.

Also in the invention, the cryopreservation composition comprises not only at least one cyclohexanediol compound, but also at least one additional cryoprotectant compound and preferably further contains of least one anti-freeze protein.

### BRIEF DESCRIPTION OF THE DRAWINGS

Figure 1 is a flow chart summarizing the cryopreservation procedure utilized in obtaining the results summarized in this application.

Figures 2-3 are plots of relative cell viability after freezing using CHD compounds in conjunction with conventional cryoprotective agents.

Figures 4-5 are plots of relative cell viability after freezing using CHD compounds in conjunction with anti-freeze proteins.

### DETAILED DESCRIPTION OF PREFERRED EMBODIMENTS

The inventors have discovered two new compounds exhibiting cryoprotective activity, 1,3-cyclohexanediol (1,3CHD) and 1,4-cyclohexanediol (1,3CHD). The inventors have also discovered that these compounds are able to work in conjunction with naturally occurring anti-freeze proteins (AFPs) to promote survival after freezing in a cumulative manner.

Cryopreservation, i.e., the preservation of cells by freezing, in the present invention may be effected in any conventional manner. By "freezing" as used herein is meant temperatures below the freezing point of water, i.e., below 0°C. Cryopreservation typically involves freezing cells to temperatures well below freezing, for example to -130°C or less. The cryopreservation temperature should be less than -20°C, more preferably -80°C or less, most preferably -130°C or less.

The cells to be cryopreserved using the CHD cryoprotectant compounds of the invention may be in suspension, may be attached to a substrate, etc., without limitation.

In the method of the invention, the cells to be protected during cryopreservation are first brought into contact with a cryopreservation composition. By being brought into contact with the cryopreservation composition is meant that the cells are made to be in contact in some manner with the cryopreservation composition so that during the reduction of temperature to the cryopreservation temperature, the cells are protected by the cryopreservation composition. For example, the cells may be brought into contact with the cryopreservation composition by filling the appropriate wells of a plate to which the cells to be protected are attached, by suspending the cells in a solution of the cryopreservation composition, etc.

The cells to be cryopreserved should also preferably be in contact with freezing compatible pH buffer comprised most typically of at least a basic salt solution, an energy source (for example, glucose) and a buffer capable of maintaining a neutral pH at cooled temperatures. Well known such materials include, for example, Dulbecco's Modified Eagle Medium (DMEM). This material may also be included as part of the cryopreservation composition.

The cryopreservation composition of the invention must contain at least one cyclohexanediol (CHD) compound, for example the cis or trans forms of 1,3-cyclohexanediol or 1,4-cyclohexanediol and racemic mixtures thereof. Preferably, the CHD compound is present in the cryopreservation composition in an amount of from, for example, 0.05 to 2.0 M, more preferably from 0.1 M to 1.0 M.

The cryopreservation composition also preferably includes a solution well suited for organ storage. The solution can include the buffers discussed above. A particular preferred solution is, for example, EuroCollins Solution comprised of dextrose, potassium phosphate monobasic and dibasic, sodium bicarbonate and potassium chloride.

In the invention, the cryopreservation composition must contains not only the CHD compound, but also at least one additional cryoprotectant compound. These additional cryoprotectant compounds is selected from the group consisting of acetamide, agarose, alginate, 1-alanine, albumin, ammonium acetate, butanediol, chondroitin sulfate, chloroform, choline, dextrans, diethylene glycol, dimethyl acetamide, dimethyl formamide, dimethyl sulfoxide (DMSO), erythritol, ethanol, ethylene glycol, formamide, glucose, glycerol, α-glycerophosphate, glycerol monoacetate, glycine, hydroxyethyl starch, inositol, lactose, magnesium chloride, magnesium sulfide, maltose, mannitol, mannose, methanol, methyl acetamide, methylformamide, methyl ureas, phenol, pluronic polyols, polyethylene glycol, polyvinylpyrrolidone, proline, propylene glycol, pyridine N-oxide, nbose, serine, sodium bromide, sodium chloride, sodium iodide, sodium nitrate, sodium sulfate, sorbitol, sucrose, trehalose, triethylene glycol trimethylamine acetate, urea, valine, xylose, etc. This additional cryoprotectant compound is preferably present in the cryopreservation composition in an amount of from, for example, 0.1 M to 10.0 M, preferably 0.1 to 2.0 M.

In a still further embodiment of the invention, the cryopreservation composition includes the CHD compound, with or without an additional cryoprotectant compound, and also includes an anti-freeze protein/peptide (AFP). AFPs also include anti-freeze glycoproteins (AFGPs) and insect anti-freeze, or "thermal hysteresis" proteins, (THPs). Naturally occurring AFPs are believed to be able to bind to the prism face of developing ice crystals, thereby altering their formation. For the fishes and insects in which these proteins occur, it means a depression of their freezing point so they are able to survive under conditions that would normally cause their body fluids to freeze.

Any of the well-known AFPs may be used in the present invention in this regard. See, for example, Sicheri and Yang, Nature, 375:427-431, (1995), describing eight such proteins. Most preferably, the AFP may be, for example, AFPI (AFP type I), AFPIII (AFP type III) and/or AFGP.

The AFPs may be present in the cryopreservation composition in an amount of from, for example, 0.01 to 1 mg/mL, more preferably 0.05 to 0.5 mg/mL, of composition, for each AFP present.

Once the cells have been contacted with the cryopreservation composition, the cells may then be frozen for cryopreservation. The cryopreservation and subsequent warming of cells may be conducted in any manner, and may utilize any additional materials, well known in the art. Preferred embodiments are described in the following discussion and the Examples set forth below.

The cooling (freezing) protocol for cryopreservation in the present invention may be any suitable type. Many types of cooling protocols are well known to practitioners in the art. Most typically, the cooling protocol calls for continuous rate cooling from the point of ice nucleation to -80°C, with the rate of cooling depending on the characteristics of the cells/tissues being frozen as understood in the art (again, see Brockbank, supra). The cooling rate may be, for example, -0.1 °C to -10°C per minute, more preferably between -1°C to -2°C per minute. Once the cells are cooled to about -80°C by this continuous rate cooling, they can be transferred to liquid nitrogen or the vapor phase of liquid nitrogen for further cooling to the cryopreservation temperature, which is below the glass transition temperature of the freezing solution (again, typically -130°C or less).

Once cryopreserved, the cells will subsequently be rewarmed for removal of the cryopreserved cells from the cryopreserved state. The warming protocol for taking the cells out of the frozen state may be any type of warming protocol, which are well known to practitioners in the art. Typically, the warming is done in a one-step procedure in which the cryopreserved specimen is placed into a water bath (temperature of about 37-42°C) until complete rewarming is effected. More rapid warming is also known.

Most preferably, the cryopreserved cells, particularly cryopreserved cells fixed to a substrate, are warmed by way of the methods described in co-pending U.S. Application No. 60/197,670 filed April 17,2001, entitled "Novel Warming Method of Cryopreserved Specimens," incorporated herein by reference in its entirety. These methods include a two-step warming protocol, with or without the use of a heat sink.

The cryopreservation composition of the present invention that includes at least one CHD compound and at least one addelinal cyoprotectant compound is surprisingly able to increase the survival of cryopreserved cells upon freezing in a cumulative manner. The following examples illustrate the surprising utility of the CHD compounds as a cryoprotectant.

### EXAMPLES

### Example 1

A primary cell strain called AV5 was used for these experiments. AV5 cells are derived from porcine heart valve leaflets. Hearts were obtained from pigs and the heart valve leaflets were then removed and washed several times with sterile phosphate-buffered saline (PBS). Small pieces (~1 mm²) were cut and placed into a 24-well microtiter plate coated with 0.2% gelatin. Dulbecco's Modified Eagle's Medium (DMEM) with 10% fetal calf serum (FCS) was added to just cover the bottom of the well and the plate was left at 37°C with 5% CO₂ in air until visible outgrowth occurred.

Outgrowth was allowed to continue until cells filled the well at which time the cells were removed from the well using trypsin and placed into a small tissue culture flask. Once the flask reached confluency, the cells were again removed with trypsin and stored in 10% dimethyl sulfoxide (DMSO) at -135°C.

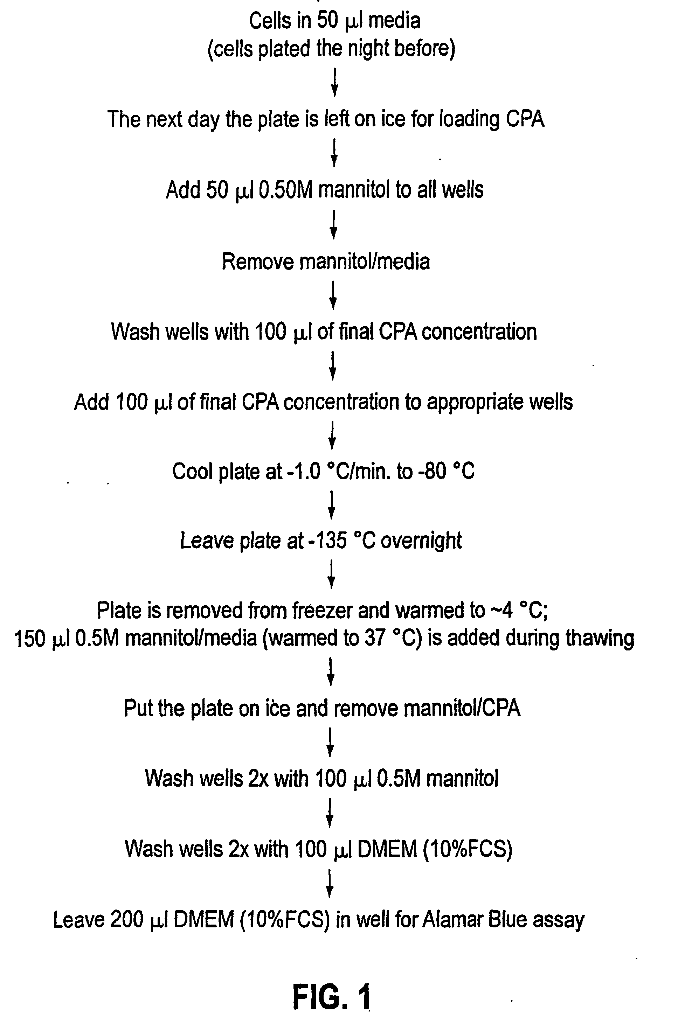

To evaluate the cryoprotective capabilities of the CHD compounds, the protocol of Figure 1 was followed. AV5 cells were plated the day before each experiment at 25,000 cells/well. At the beginning of each experiment, the plate was placed on ice and the cells were exposed to mannitol prior to loading the various cryopreservation compositions.

All of the cryopreservation compositions were formulated in EuroCollins Solution, consisting of dextrose, potassium phosphate monobasic and dibasic, sodium bicarbonate and potassium chloride. The plates were then cooled at the rate of -1.0°C/min to -80°C, and then further cooled with liquid nitrogen vapor and stored overnight at -135°C.

The next day the plate was removed and warmed to ~4°C at which point it was put back on ice. During warming, 150 µl of 0.5 M mannitol in cell culture media was added to the cells. Once back on ice, the cryoprotectant/mannitol mixture was removed. The cells were washed twice with 0.5 M mannitol/media and twice with DMEM (10%FCS).

Cell viability was then determined using the non-invasive metabolic indicator Alamar Blue (Trek Diagnostics). Alamar Blue is a fluorescent dye that measures the oxidation/reduction reactions within cells, and thus is indicative of the overall viability of the cells after exposure to cryoprotective agents. A volume of 20 µl Alamar Blue was added to cells in 200 µl of DMEM (10%FCS) and the plate was incubated at 37°C for 3 hours. Fluorescence from Alamar Blue was read in a fluorescent microplate reader (Fmax fluorescent microplate reader by Molecular Dynamics) using an excitation wavelength of 544 nm and an emission wavelength of 590 nm.

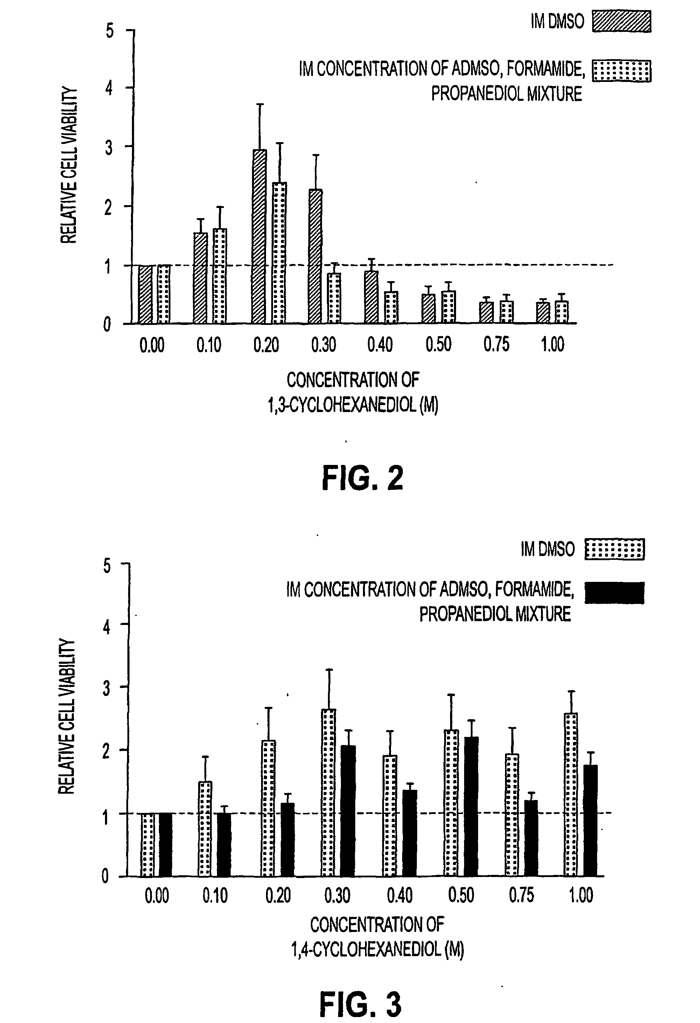

The first set of experiments involved using two CPA compositions, either 1 M dimethyl sulfoxide (DMSO) (left bar of graph at each concentration of 1,3CHD in Figure 2 and 1,4CHD in Figure 3) or a combination of DMSO, formamide and propanediol at a final concentration of 1 M (right bar of graph at each concentration of 1,3CHD in Figure 2 and 1,4CHD in Figure 3). Concentrations varying from 0 to 1 M of 1,3CHD and 0 to 1 M 1,4CHD were added to these two separate CPA compositions for additional experiments. Cell viability was assessed using the assay described above.

The results are graphically summarized in Figures 2 and 3. Figure 2 relates to 1,3CHD, while Figure 3 relates to 1,4CHD. For each, the data was normalized to the conventional cryoprotectant alone and is the mean (+/- SEM) of 12 replicates. As shown in these two Figures, in the presence of varying concentrations of both CHD molecules, viability was significantly increased over the comparative cryopreservation compositions that contained only the conventional cryoprotectants without a CHD compound (i.e., concentration of 0.00 CHD).

Similar results to the foregoing for AV5 have been obtained with other cell types, including (1) A10, an established cell line of smooth muscle cells derived from rat thoracic aorta and (2) J15, a primary cell strain of smooth muscle cells derived from rabbit jugular veins.

### Example 2

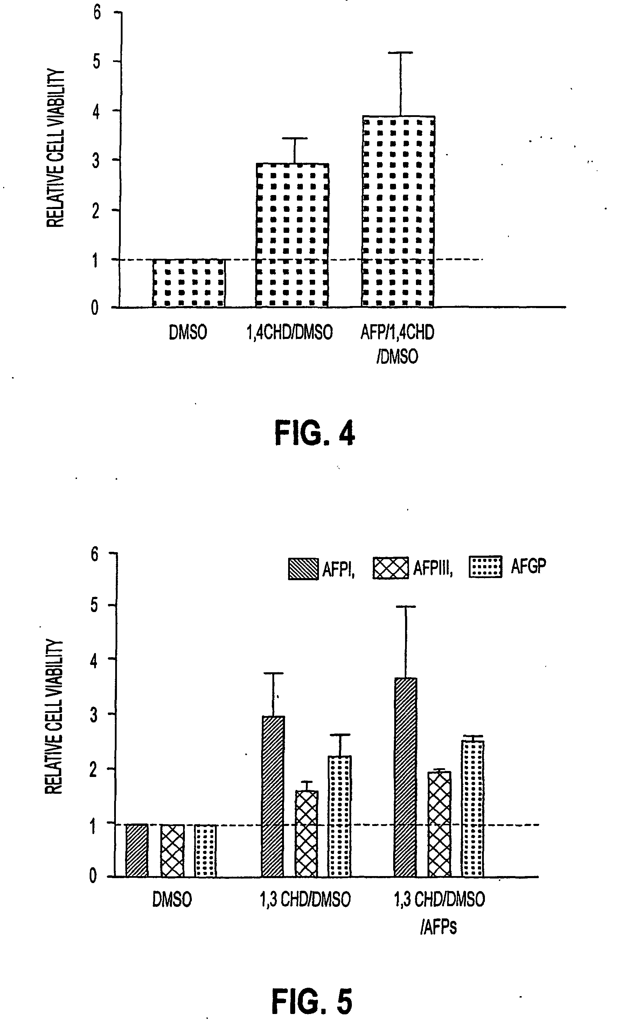

In this Example, an anti-freeze protein (AFP) was added to the cryoprotective composition. Varying concentrations of three different AFPs (AFPI, AFPIII and AFGP) were used along with 1 M DMSO and either 0.25 M 1,3CHD (Figure 5) or 0.5 M 1,4CHD (Figure 4). Again, the protocol of Figure 1 was followed. The data were normalized to the comparative conventional cryoprotectant alone (i.e., DMSO alone), and the results are presented in Figures 4 and 5.

In Figure 4, the relative cell viability of AV5 cells after freezing using a combination of 1,4CHD/AFPI/DMSO is summarized. Concentrations of the constituents included 0.5 M 1,4CHD, 0.1 mg/mL AFPI and 1 M DMSO. The graph depicts viability in the presence of 1 M DMSO alone or in combination with 1,4CHD or 1,4CHD plus AFPI at the above concentrations. Data was normalized to the conventional cryoprotectant (DMSO) alone and is the mean (+/- SEM) of 3 replicates.

In Figure 4, the results demonstrated the increased viability of the cells upon addition of the CHD molecule as compared to the conventional cryoprotectant alone and a further increase in viability upon addition of the AFPI protein to the conventional cryoprotectant/CHD mixture. Thus, a cumulative effect in the presence of AFP and CHD was demonstrated.

In Figure 5, the relative cell viability of AV5 cells after freezing using a combination of 1,3CHD, DMSO and three different AFP proteins is summarized. Concentrations of the constituents included 0.25 M 1,3CHD, 1 M DMSO and 0.1 mg/mL for each AFP protein (left bar of graph is with AFPI, middle bar of graph is with AFPIII, and right bar of graph is with AFGP). Data was normalized to the DMSO alone and is the mean (+/- SEM) of 3 replicates.

From the data of Figure 5, AFPI appears to confer the best protection to the cells in this Example as observed by an increase in cell viability as compared to the conventional cryoprotectant alone or to the conventional cryoprotectant/CHD mixture. This cumulative increase in viability is not observed in the presence of the AFPs plus a conventional cryoprotectant alone (i.e., without the CHD present).

While this invention has been described in conjunction with specific embodiments thereof, it is evident that many alternatives, modifications and variations will be apparent to those skilled in the art. Accordingly, the preferred embodiments of the invention as set forth herein are intended to be illustrative only, and not limiting. Various changes may be made without departing from the scope of the invention as defined in the following claims.

## Claims

1. A method of cryopreserving cells, comprising
bringing the cells into contact with a cryopreservation composition containing at least one cyclohexanediol compound selected from the group consisting of the cis or trans forms of 1,3-cyclohexanediol and 1,4-cyclohexanediol, and racemic mixtures thereof and at least one additional cryoprotectant compound, wherein the at least one additional cryoprotectant compound is selected from the group consisting of acetamide, agarose, alginate, 1-alanine albumin, ammonium acetate, butanediol, chondroitin sulfate, chloroform, choline, dextrans, diethylene glycol, dimethyl acetamide, dimethyl formamide, dimethyl sulfoxide (DMSO), erythritol, ethanol, ethylene glycol, formamide, glucose, glycerol, α-glycerophosphate, glycerol monoacetate, glycine, hydroxyethyl starch, inositol; lactose, magnesium chloride, magnesium sulfate, maltose, mannitol, mannose, methanol, methyl acetamide, methylformamide, methyl ureas, phenol, pluronic polyols, polyethylene glycol, polyvinylpyrrolidone, proline, propylene glycol, pyridine N-oxide, ribose, serine, sodium bromide, sodium chloride, sodium iodide, sodium nitrate, sodium sulfate, sorbitol, sucrose, trehalose, triethylene glycol, trimethylamine acetate, urea, valine and xylose, and
subsequently reducing the temperature of the cells to a cryopreservation temperature.

2. A method according to claim 1, wherein the cryopreservation composition further contains at least one anti-freeze protein.

3. A method according to claim 2, wherein the at least one anti-freeze protein is an anti-freeze glycoprotein.

4. A method according to claim 1, wherein the cryopreservation temperature is -20°C or less.

5. A cryopreservation composition comprising at least one cyclohexanediol compound and at least one additional cryoprotectant compound,
wherein the at least one cyclohexanediol compound is selected from the group consisting of the cis or trans forms of 1,3-cyclohexanediol and 1,4-cyclohexanediol, and racemic mixtures thereof, and
wherein the at least one additional cryoprotectant compound is selected from the group consisting of acetamide, agarose, alginate, 1-alanine, albumin, ammonium acetate, butanediol, chondroitin sulfate, chloroform, choline, dextrans, diethylene glycol, dimethyl acetamide, diinethyl formamide, dimethyl sulfoxide (DMSO), erythritol, ethanol, ethylene glycol, formamide, glucose, glycerol, α-glycerophosphate, glycerol monoacetate, glycine, hydroxyethyl starch, inositol, lactose, magnesium chloride, magnesium sulfate, maltose, mannitol, mannose, methanol, methyl acetamide, methylformamide, methyl ureas, phenol, pluronic polyols, polyethylene glycol, polyvinylpyrrolidone, proline, propylene glycol, pyridine N-oxide, ribose, serine, sodium bromide, sodium chloride, sodium iodide, sodium nitrate, sodium sulfate, sorbitol, sucrose, trehalose, triethylene glycol, trimethylamine acetate, urea, valine and xylose.

6. A cryopreservation composition according to claim 5, wherein the cryopreservation composition further contains at least one anti-freeze protein.

7. A cryopreservation composition according to claim 6, wherein the at least one anti-freeze protein is an anti-freeze glycoprotein.

## Patentansprüche

1. Verfahren zum Kältekonservieren von Zellen, das Folgendes aufweist, nämlich Inberührungbringen der Zellen mit einer Kältekonservierungszusammensetzung, die zumindest eine Cyclohexanediolverbindung, ausgewählt aus der Gruppe bestehend aus den *cis-* oder *trans*-Formen von 1,3-Cyclohexandiol und 1,4-Cyclohexandiol und racemischen Mischungen davon und zumindest eine zusätzliche Kälteschutzmittelverbindung aufweist, wobei die zumindest eine zusätzliche Kälteschutzmittelverbindung ausgewählt ist aus der Gruppe bestehend aus Acetamid, Agarose, Alginat, L-Alanin, Albumin, Ammoniumacetat, Butandiol, Chondroitinsulfat, Chloroform, Cholin, Dextranen, Diethylenglykol, Dimethylacetamid, Dimethylformamid, Dimethylsulfoxid (DMSO), Erythritol, Ethanol, Ethylenglykol, Formamid, Glukose, Glycerin, α-Glycerinphosphat, Glycerinmonoacetat, Glycin, Hydroxyethylstärke, Inositol, Lactose, Magnesiumchlorid, Magnesiumsulfat, Maltose, Mannitol, Mannose, Methanol, Methylacetamid, Methylformamid, Methylharnstoffen, Phenol, pluronischen Polyolen, Polyethylenglykol, Polyvinylpyrrolidon, Prolin, Propylenglykol, Pyridin-N-oxid, Ribose, Serin, Natriumbromid, Natriumchlorid, Natriumjodid, Natriumnitrat, Natriumsulfat, Sorbit, Saccharose, Trehalose, Triethylenglykol, Trimethylaminacetat, Harnstoff, Valin und Xylose, und
anschließend Absenken der Temperatur der Zellen auf eine Kältekonservierungstemperatur.

2. Verfahren nach Anspruch 1, wobei die Kältekonservierungszusammensetzung weiterhin zumindest ein Anti-Frost-Protein aufweist.

3. Verfahren nach Anspruch 2, wobei das zumindest eine Anti-Frost-Protein ein Anti-Frost-Glycoprotein ist.

4. Verfahren nach Anspruch 1, wobei die Kältekonservierungstemperatur -20°C oder weniger beträgt.

5. Kältekonservierungszusammensetzung, die zumindest eine Cyclohexandiolverbindung und zumindest eine zusätzliche Kälteschutzmittelverbindung aufweist, wobei die zumindest eine Cyclohexandiolverbindung ausgewählt ist aus der Gruppe bestehend aus den *cis-* oder *trans-*Formen von 1,3-Cyclohexandiol und 1,4-Cyclohexandiol und racemischen Mischungen davon, und wobei die zumindest eine zusätzliche Kälteschutzmittelverbindung ausgewählt ist aus der Gruppe bestehend aus Acetamid, Agarose, Alginat, L-Alanin, Albumin, Ammoniumacetat, Butandiol, Chondroitinsulfat, Chloroform, Cholin, Dextranen, Diethylenglykol, Dimethylacetamid, Dimethylformamid, Dimethylsulfoxid (DMSO), Erythritol, Ethanol, Ethylenglykol, Formamid, Glukose, Glycerin, α-Glycerinphosphat, Glycerinmonoacetat, Glycin, Hydroxyethylstärke, Inositol, Lactose, Magnesiumchlorid, Magnesiumsulfat, Maltose, Mannitol, Mannose, Methanol, Methylacetamid, Methylformamid, Methylharnstoffen, Phenol, pluronischen Polyolen, Polyethylenglykol, Polyvinylpyrrolidon, Prolin, Propylenglykol, Pyridin-N-oxid, Ribose, Serin, Natriumbromid, Natriumchlorid, Natriumjodid, Natriumnitrat, Natriumsulfat, Sorbit, Saccharose, Trehalose, Triethylenglykol, Trimethylaminacetat, Harnstoff, Valin und Xylose.

6. Kältekonservierungszusammensetzung nach Anspruch 5, wobei die Kältekonservierungszusammensetzung weiterhin zumindest ein Anti-Frost-Protein aufweist.

7. Kältekonservierungszusammensetzung nach Anspruch 6, wobei das zumindest eine Anti-Frost-Protein ein Anti-Frost-Glycoprotein ist.

## Revendications

1. Procédé de cryoconservation des cellules, comprenant les étapes consistant à
mettre les cellules en contact avec une composition de cryoconservation contenant au moins un composé cyclohexanediol choisi dans le groupe comprenant les formes cis et trans de 1,3-cyclohexanediol et 1,4-cyclohexanediol et les mélanges racémiques de ceux-ci et au moins un composé cryoprotecteur supplémentaire, dans lequel le au moins un composé cryoprotecteur supplémentaire est choisi dans le groupe comprenant l'acétamide, l'agarose, l'alginate, la 1-alanine, l'albumine, l'acétate d'ammonium, le butanediol, le sulfate de chondroitine, le chloroforme, la choline, les dextranes, le diéthylène glycol, le diméthylacétamide, le diméthylformamide, le diméthylsulfoxide (DMSO), l'erythritol, l'éthanol, l'éthylène glycol, le formamide, le glucose, le glycérol, l'α-glycérophosphate, le monoacétate de glycérine, la glycine, l'hydroxyéthylamidon, l'inositol, le lactose, le chlorure de magnésium, le sulfate de magnésium, le maltose, le mannitol, le mannose, le méthanol, le méthylacétamide, le méthylformamide, les methyl-urées, le phénol, les polyols pluroniques, le polyéthylène glycol, le polyvinylpyrrolidone, le proline, le propylène glycol, la pyridine N-oxyde, la ribose, la sérine, le bromure de sodium, le chlorure de sodium, l'iodure de sodium, le nitrate de sodium, le sulfate de sodium, le sorbitol, le sucrose, le tréhalose, le triéthylène glycol, l'acétate de triméthylamine, l'urée, la valine et la xylose, et
par la suite, abaisser la température des cellules à une température de cryoconservation.

2. Procédé selon la revendication 1, dans lequel la composition de cryoconservation contient en outre au moins une protéine antigel.

3. Procédé selon la revendication 2, dans lequel ladite au moins une protéine antigel est une glycoprotéine antigel.

4. Procédé selon la revendication 1, dans lequel la température de cryoconservation est inférieure ou égale à -20 °C.

5. Composition de cryoconservation comprenant au moins un composé cyclohexanediol et au moins un composé cryoprotecteur supplémentaire,
dans laquelle ledit au moins un composé cyclohexanediol est choisi dans le groupe comprenant les formes cis et trans de 1,3-cyclohexanediol et 1,4-cyclohexanediol et les mélanges racémiques de ceux-ci, et
dans laquelle ledit au moins un composé cryoprotecteur supplémentaire est choisi dans le groupe comprenant l'acétamide, l'agarose, l'alginate, la 1-alanine, l'albumine, l'acétate d'ammonium, le butanediol, le sulfate de chondroïtine, le chloroforme, la choline, les dextranes, le diéthylène glycol, le diméthylacétamide, le diméthylformamide, le diméthylsulfoxide (DMSO), l'erythritol, l'éthanol, l'éthylène glycol, le formamide, le glucose, le glycérol, l' α-glycérophosphate, le monoacétate de glycérine, la glycine, l'hydroxyéthylamidon, l'inositol, le lactose, le chlorure de magnésium, le sulfate de magnésium, le maltose, le mannitol, le mannose, le méthanol, le méthylacétamide, le méthylformamide, les methyl-urées, le phénol, les polyols pluroniques, le polyéthylène glycol, le polyvinylpyrrolidone, le proline, le propylène glycol, la pyridine N-oxyde, la ribose, la sérine, le bromure de sodium, le chlorure de sodium, l'iodure de sodium, le nitrate de sodium, le sulfate de sodium, le sorbitol, le sucrose, le tréhalose, le triéthylène glycol, l'acétate de triméthylamine, l'urée, la valine et la xylose.

6. Composition de cryoconservation selon la revendication 5, dans laquelle la composition de cryoconservation contient en outre au moins une protéine antigel.

7. Composition de cryoconservation selon la revendication 6, dans laquelle ladite au moins une protéine antigel est une glycoprotéine antigel.
